(19) **Europäisches Patentamt European Patent Office Office européen des brevets**

(11) **EP 4 760 221 A1**

(12) **EUROPEAN PATENT APPLICATION**

(43) Date of publication:
17.06.2026 Bulletin 2026/25

(21) Application number: 24219121.1

(22) Date of filing: 11.12.2024

(51) International Patent Classification (IPC):
*G01J 3/02* (2006.01) *G01J 3/453* (2006.01)
*G01B 9/02091* (2022.01)

(52) Cooperative Patent Classification (CPC):
**G01J 3/4531; G01J 3/0218; G01J 3/0256;
G01J 3/4532**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Imec VZW**
**3001 Leuven (BE)**

(72) Inventors:
• **KIM, Joonyoung**
**3000 Leuven (BE)**

• **YURTSEVER, Gunay**
**3511 Hasselt (BE)**
• **VAN DORPE, Pol**
**3510 Spalbeek (BE)**
• **ROTTENBERG, Xavier**
**3010 Kessel-Lo (BE)**
• **SUBRAMANIAN, Ananth**
**9030 Mariakerke (BE)**

(74) Representative: **AWA Sweden AB**
**Matrosgatan 1**
**Box 5117**
**200 71 Malmö (SE)**

(54) **A DEVICE AND A METHOD FOR FOURIER TRANSFORM SPECTROSCOPY AND A SYSTEM FOR SPECTRAL DOMAIN INTERFEROMETRIC MEASUREMENT**

(57) A device (100; 200; 300) for Fourier Transform spectroscopy comprises: a first light propagating unit (120; 220; 320) receiving a first portion of a light signal and being configured to couple out fractions of the first portion by a sequence of first out-coupling elements (124; 224; 324) with a remaining fraction of the first portion continuing propagation along the first waveguide (122; 222; 322); a second light propagating unit (130; 230; 330) receiving a second portion of the light signal and being configured to couple out fractions of the second portion by a sequence of second out-coupling elements (132; 232; 332) with a remaining fraction of the second portion of the light signal continuing propagation along the second waveguide (132; 232; 332); wherein optical path lengths between sequential out-coupling elements are different in the second waveguide (132; 232; 332) and the first waveguide (122; 232; 322).

Fig. 3a

Processed by Luminess, 75001 PARIS (FR)

(Cont. next page)

Fig. 3b

## Description

Technical field

The present description relates to Fourier Transform spectroscopy. In particular, the present description relates to a device for Fourier Transform spectroscopy and a method for guiding a light signal for Fourier Transform spectroscopy. The present description also relates to a system for Fourier Transform spectroscopy and a system for spectral domain interferometric measurement.

Background

An optical spectrometer is an instrument used for measuring properties of a light signal in a range of the electromagnetic spectrum. The optical spectrometer may be used for characterizing a sample, such as for determining compounds present in a sample.

A Fourier Transform (FT) spectrometer is configured to use interference to determine spectral content of the light signal. In the FT spectrometer, the light signal is split and configured to propagate along different paths. An interference based on portions of light signals having propagated along different optical path lengths is detected. This is performed for multiple sets of different optical path lengths providing a FT of the light signal to be detected.

FT spectrometry may provide a high signal-to-noise ratio and may provide high resolution over a large range, making FT spectrometry an interesting technology for spectrometry.

However, the FT spectrometer requires interference for a plurality of different path length relations of the portions of light signals to be detected. Using a Fourier Transform Spatial Heterodyne Spectrometer (FT-SHS), detection of interference for the plurality of different path length relations may be performed simultaneously. However, the FT-SHS uses a plurality of Mach-Zehnder Interferometers (MZIs). Thus, the light signal is split to a plurality of MZIs and each MZI is used for detecting interference of portions of the light signal based on a unique path length relation between the portions of the light signal. The use of multiple MZIs, at least some of which having relatively long delay lines, imply that the FT-SHS requires a large area, in particular if a high resolution and large bandwidth is desired. Also, relatively high losses in the light signal may be introduced due to the need of multiple splitting of the light signal.

Summary

An objective of the present description is to facilitate a compact detection for Fourier Transform spectroscopy. A further objective of the present description is to facilitate fast detection of spectral information and to facilitate detection of spectral information with low losses of a light signal to be detected.

These and other objectives are at least partly met by the invention as defined in the independent claims. Preferred embodiments are set out in the dependent claims.

According to a first aspect, there is provided a device for Fourier Transform (FT) spectroscopy, said device comprising: an input waveguide for receiving a light signal; a splitter connected to the input waveguide for splitting the light signal into a first portion and a second portion; a first light propagating unit configured to receive the first portion of the light signal, wherein the first light propagating unit comprises a first waveguide configured to propagate the first portion of the light signal, and a sequence of first out-coupling elements, wherein the first out-coupling elements are spaced apart along an extension of the first waveguide, wherein first out-coupling elements in the sequence are configured to couple a fraction of the first portion of the light signal out of the first waveguide with a remaining fraction of the first portion of the light signal continuing propagation along the first waveguide; a second light propagating unit configured to receive the second portion of the light signal, wherein the second light propagating unit comprises a second waveguide configured to propagate the second portion of the light signal, and a sequence of second out-coupling elements, wherein the second out-coupling elements are spaced apart along an extension of the second waveguide, wherein second out-coupling elements in the sequence are configured to couple a fraction of the second portion of the light signal out of the second waveguide with a remaining fraction of the second portion of the light signal continuing propagation along the second waveguide; wherein an optical path length along the second waveguide between sequential second out-coupling elements is different than an optical path length along the first waveguide between corresponding sequential first out-coupling elements, wherein the first out-coupling elements and second out-coupling elements form a sequence of pairs, each comprising one second out-coupling element and one corresponding first out-coupling element, wherein a difference in the optical path length propagated by the fraction of the second portion of the light signal out-coupled by the second out-coupling element compared to the optical path length propagated by the fraction of the first portion of the light signal out-coupled by the corresponding first out-coupling element is configured to be different along the sequence of pairs.

Thanks to the device, multiple out-coupling elements are arranged in each of a first light propagating unit and a second light propagating unit. This implies that a difference in optical path length provided for a first pair of light-coupling elements may also be utilized by following pairs of light-coupling elements. Hence, the difference in optical path length for a particular pair of out-coupling elements may be formed by a sum of differences in the sequence of pairs.

Each pair of out-coupling elements is configured to provide a fraction of the first portion of the light signal and a fraction of the second portion of the light signal for forming a combined signal by interference of the out-coupled light. This combined signal may be referred to as an interferential signal.

The combined signal may be combined within the device. However, it should be realized that the light out-coupled from the out-coupling elements may be directed towards a common location, which may be external to the device, for combination in such external location.

This provides an efficient manner of defining a plurality of different optical path length relations such that a compact arrangement of the light propagating units is provided. Hence, a small area for providing the differences in optical path lengths may be used, such that a compact device may be provided.

In particular, the device does not require movable parts to be used for defining different optical path length relations. This implies that the device may not need to be combined with bulky components.

The device may be seen as a sequence of cascaded interferometers since the optical path length difference for a pair of out-coupling elements is passed on to following pairs in the sequence. The use of cascaded interferometers provides a compact arrangement of the path length differences needed.

Further, thanks to the out-coupling elements being configured to out-couple a fraction of the portion of the light signal and allow a remaining fraction to pass along the waveguide, out-coupling elements in the sequence may simultaneously out-couple light for detection. This implies that the interference corresponding to different path length relations may be simultaneously detected. This implies that interference signals for allowing detection of FT spectroscopy may be simultaneously formed and hence simultaneously detected. Thus, the device facilitates acquiring information for FT spectroscopy in a fast manner.

The device may in particular be suited for an on-chip arrangement as a very compact device may be provided. Thus, the waveguides of the device may be provided on a common substrate. According to an embodiment, the device may be formed as a photonic integrated circuit (PIC). The PIC is an integrated device wherein two or more photonic components form a functioning circuit for transporting and/or processing optical signals. This may be a particularly suitable manner of providing a compact device.

Further, since the optical path length relations are not defined in completely separate interferometers, relative phase errors between the interferometers may be reduced or minimized. This ensures that high accuracy of forming the interference signals may be provided.

In addition, a spectral resolution may be improved by increasing a maximum optical path length difference being used for forming interference signals.

The device may allow a large optical path length difference to be used while having a compact device. Thus, the device may be configured to provide a high spectral resolution.

The device provides interference signal to be formed based on different optical path length relations between the first and second portions of the light signals. The different interference signals thus extract information for different wavelengths (frequencies) in the light signal. This information is provided in different spatial positions. Thus, the device may be referred to as a Fourier Transform Spatial Heterodyne Spectrometer (FT-SHS).

It should be realized that the light signal may relate to light in a visible part of the electromagnetic spectrum, but the light signal may also or alternatively comprise wavelengths outside visible light, such as ultraviolet light or infrared light.

Each waveguide, i.e., the input waveguide, the first waveguide and the second waveguide, may be any structure providing guiding of the light signal or the first or second portion of the light signal, respectively. The waveguide may be configured to confine light in a cross-section perpendicular to an extension of the waveguide. The waveguide may thus be configured to restrict light to follow a path defined by the extension of the waveguide, whereby transmission of light with low loss may be provided. The waveguide may for instance guide light by light being reflected on inner walls of the waveguide by total internal reflection or by a reflective coating being provided on the walls of the waveguide. The waveguide is configured to propagate the first optical signal along the path of the waveguide.

The waveguides may be formed by any suitable core material surrounded by a suitable cladding material. Thus, the light may be propagated in the core material and may be reflected by total internal reflection in an interface between the core material and the cladding material. Thus, the core material may have a lower refractive index than the cladding material. The core material may be any suitable material for propagating the light with low loss, i.e., having low absorption in the wavelength range of light. For instance, the waveguides may be formed by a core material of silicon or silicon nitride surrounded by a cladding material of silicon dioxide. However, it should be realized that other materials may be used.

A cross-section of the waveguide may be rectangular. However, it should be realized that other shapes of the waveguides may be used, such as a ridge waveguide.

The splitter may be any structure configured to provide a splitting of the light signal into a first and a second portion. The splitter may be configured to split light equally into the first portion and the second portion, such that each of the first light propagating unit and the second propagation unit receives 50% of the light signal (50/50 splitter). However, it should be realized that other proportions in splitting the light signal may be used, such that a larger intensity is provided to the first light propagating unit or to the second light propagation unit.

The splitter may be formed by a directional coupler for diverting a portion of the light signal from the input waveguide. The splitter may alternatively be formed by a multi-mode interference coupler for forming the first and second portions of the light signal. It should be realized that the splitter may also be implemented in another manner.

The first and second sequences of out-coupling elements are arranged in different locations along the extension of the first waveguide and the second waveguide, respectively. Thus, the out-coupling elements form a sequence in that the portion of the light signal propagated in the first and the second waveguide, respectively, sequentially reaches the out-coupling elements, one after another.

It should further be realized that the waveguides may be configured to extend in any suitable path. Thus, the waveguides need not be arranged along a straight path. Thus, the out-coupling elements being spaced apart along an extension of the waveguide implies that each out-coupling element is associated with a location in the waveguide and sequential out-coupling elements are spaced apart by a path length along the waveguide between the out-coupling elements. This does not necessarily correspond to a shortest physical distance between the out-coupling elements since the waveguides need not extend along a straight path.

In particular, at least one of the first and second waveguides may be arranged to extend along a curved path in order to provide a difference in optical path length between the sequential second out-coupling elements and corresponding sequential first out-coupling elements.

The first waveguide may be configured to extend in a first waveguide layer of the device. Thus, the first waveguide may be arranged in a layer which may be parallel to a substrate. This may facilitate forming of the first waveguide in an integrated device.

The second waveguide may be configured to extend in a second waveguide layer of the device. Thus, the second waveguide may also be arranged in a layer which may be parallel to a substrate. This may facilitate forming of the second waveguide in an integrated device. It should be realized that the first and second waveguides may be formed in a common waveguide layer or may be formed in separate waveguide layers, with one waveguide layer arranged above the other waveguide layer.

The first and second out-coupling elements may be formed by any structure allowing a fraction of the portion of the light signal to be out-coupled from the first and second waveguides, respectively, and a remaining fraction to continue propagation in the first and second waveguides, respectively. Thus, the first and second out-coupling elements may be configured to divert a fraction of the portion of the light signal out of the first and second waveguides, respectively. This may be achieved, for example, using a grating coupler or a directional coupler. The first and second out-coupling elements may also or alternatively be configured to use an evanescent field of the portion of the light signal propagating in the first and second waveguide, respectively, for out-coupling the fraction of the portion of the light signal into another waveguide being arranged in close vicinity to the first or second waveguide, respectively.

It should be realized that a last out-coupling element in the sequence need not be configured to out-couple a fraction of light. Rather, the last out-coupling element may be configured to provide all remaining light for forming the interference signal. In fact, the device may be configured to provide a combination of the remaining fraction of the first portion of the light signal and the remaining fraction of the second portion of the light signal after having passed all out-coupling elements. This may be achieved not necessarily using an out-coupling element but rather by directly combining the remaining fractions of the first and second portions of the light signals.

The optical path length along the first and second waveguide, respectively, between sequential out-coupling elements may be defined by several factors, including a shape of a cross-section of the waveguide, a refractive index of the waveguide, and a physical length along the waveguide. The difference in path length of the first and second waveguides between the sequential first out-coupling elements and the sequential second out-coupling elements, respectively, may be provided by different physical lengths of the waveguides between the out-coupling elements. However, it should be realized that the path length difference may be provided in other manners, such as using different refractive indices (e.g., different materials) or different shapes of the cross-section of the waveguides.

It should be realized that the path length difference may be provided by a structure of the waveguides. Thus, the path length differences may be provided by a passive optical set-up such that no active control of the device may be necessary. However, it should be realized that the device may alternatively comprise tuning elements for tuning an optical path length along at least one of the first waveguide or the second waveguide. Such tuning element may be achieved using thermo-optic or electro-optic effect to change refractive index of the waveguide.

The sequence of pairs is formed by the sequence of first and second out-coupling elements. Thus, each pair is defined by a second out-coupling element and a corresponding first out-coupling element. This implies that the out-coupling elements in a pair have a common order in a respective sequence. Hence, a first pair is defined by a first one of the second out-coupling elements and a first one of the first out-coupling element, a second pair is defined by a second one of the second out-coupling elements and a second one of the first out-coupling element, and so on.

There is a difference in optical path length in the first and second waveguides between each of the pairs in the sequence. This implies that an optical path length difference is added between each pair such that the interference signal that can be formed by each pair provides a unique path length difference between the first portion of the light signal and the second portion of the light signal.

It should be realized that the added difference in optical path length between sequential pairs may be the same throughout the sequence. However, it should be realized that different path length differences may be provided between sequential pairs. The optical path length along the second waveguide between sequential second out-coupling elements may be larger than an optical path length along the first waveguide between corresponding sequential first out-coupling elements. However, it should be realized that for some path length differences in the sequence, the optical path length along the first waveguide between sequential first out-coupling elements may instead be larger than an optical path length along the second waveguide between corresponding sequential second out-coupling elements. This may not be the most efficient manner of using the lengths of the waveguides, but it may still be possible to form interferential signals for FT spectroscopy in such manner.

It should be realized that the device for FT spectroscopy according to the first aspect may not actually perform detection of light for spectroscopy. Rather, the device at least provides out-coupling of fractions of the first portion and the second portion of the light signal, allowing interference signals to be formed which may further be detected for performing spectroscopy. However, it should be realized that the detectors may be arranged in a separate physical unit and that light may be out-coupled by the device for being detected in a separate detector unit. However, according to an embodiment, the device further comprises photo-sensitive elements for detecting light. This may be used for providing a compact spectrometer.

According to an embodiment, there may be provided a spectrometer configured to receive a light signal carrying information, such as carrying information from a sample, and a device according to the first aspect. Thus, the light signal received by the input waveguide of the device may be the light signal carrying information, such as information from the sample. The light signal may for instance be a light signal representing light transmission through a sample or light reflection from a sample.

The spectrometer may further comprise a plurality of photo-sensitive elements configured to detect the combined signals formed by output from each pair of out-coupling elements. The combined signals may be detected in different spatial positions and the spectrometer may thus be considered to detect information in "spatial" domain. The detected information from the photo-sensitive elements may be converted into spectral domain by performing a Fourier Transform on the spatial domain information.

According to an embodiment, the first out-coupling elements in the sequence of first out-coupling elements are configured to couple out an increasing fraction of the light signal from the first waveguide in the sequence, and wherein the second out-coupling elements in the sequence of second out-coupling elements are configured to couple out an increasing fraction of the light signal from the second waveguide in the sequence.

This implies that an out-coupling ratio provided by the out-coupling elements increases along the sequence of out-coupling elements.

It should be realized that the first one of the first out-coupling elements in the sequence receives an entire first portion of the light signal. Thus, by having a relatively small out-coupling ratio, a small fraction is out-coupled and a relatively large remaining fraction continues propagation. Then, the second one of the first out-coupling elements receives the remaining fraction of the first portion of the light signal after the out-coupling in the first one of the first out-coupling elements. Hence, the second one of the first out-coupling elements may have a larger out-coupling ratio than the first one of the first out-coupling elements such that an optical power output by each of the out-coupling elements may be equal. The same applies to the second out-coupling elements.

While it would be preferred that an optical power output by each of the out-coupling elements is equal, (slight) differences in the optical power may be taken into account in processing of measurements. For instance, calibration may be used for handling differences in optical power being output.

The out-coupling elements being configured to couple out an increasing fraction in the sequence implies that the out-coupling elements may be configured to couple out an increasing fraction of the signal received by the out-coupling element. In other words, the out-coupling elements provide an increasing out-coupling ratio in the sequence. Thus, a late out-coupling element being arranged after an early out-coupling element in the sequence has a larger out-coupling ratio than the early out-coupling element.

Thus, thanks to the out-coupling elements being configured to couple out an increasing fraction in the sequence, the optical power output by each of the out-coupling elements may be the same. This implies that information relating to different optical path length differences may be acquired with similar optical power, facilitating processing of detected signals.

According to an embodiment, the optical path length along the second waveguide between sequential second out-coupling elements is larger than the optical path length along the first waveguide between corresponding sequential first out-coupling elements, wherein the difference in the optical path length propagated by the fraction of the second portion of the light signal out-coupled by the second out-coupling element compared to the optical path length propagated by the fraction of the first portion of the light signal out-coupled by the corresponding first out-coupling element is configured to increase along the sequence of pairs.

This implies that an optical path length difference is added between each pair in the sequence. Hence, the optical path length difference provided in a pair is a sum of added path length differences along the sequence. This implies that a compact arrangement of the first and second waveguides may be provided, since the optical path length difference is monotonically increasing along the pairs in the sequence.

It should be realized that the optical path length difference provided by the pairs of the sequence may form a non-monotonically increasing function along the sequence. However, this would require longer waveguides for achieving the same path length differences compared to a monotonically increasing function.

According to an embodiment, the differences in optical path lengths is configured to increase along the sequence of pairs by an equal added path length between sequential pairs.

This implies that the device provides interference signals which may relate to equally spaced differences in optical path lengths. This may facilitate processing of detected interference signals for determining the spectral information in the light signal.

It should be realized that due to imperfections in fabrication, the first and second waveguides may not be designed to provide exactly equally added path lengths between sequential pairs.

The device may comprise tuning elements for providing a tuning of the optical path length of the first waveguide and/or the second waveguide. A tuning element may be provided between each pair of out-coupling elements for tuning the added path length. The tuning elements may be used for correcting any deviations in added path lengths from a desired or nominal value. Thus, the tuning elements may be controlled based on calibration for ensuring that equal added path lengths are provided between sequential pairs.

According to an embodiment, out-coupling of the fraction of the first portion of the light signal and out-coupling of the fraction of the second portion of the light signal by each pair of first and second out-coupling elements is configured to be received directly in a common detection waveguide.

Thus, the device may comprise a common detection waveguide associated with each pair of out-coupling elements. The out-coupling elements may be provided by the first and second waveguide, respectively, being arranged sufficiently close to the common detection waveguide for allowing the fraction of the portion of the first and the second light signal, respectively, to be out-coupled through an evanescent field.

This implies that the interference signal may be formed directly in the common detection waveguide providing a very compact arrangement of the device for combining the out-coupled fractions of light. The first and second waveguides may be arranged to follow a path such that the first and second waveguides only are in close vicinity to the common detection waveguide in locations of the out-coupling elements so as to form an out-coupling element by the close arrangement of the waveguides to each other.

Separate common detection waveguides may be formed for each pair of out-coupling elements. The detection waveguide is called a common detection waveguide in that it receives out-coupled light from both the first out-coupling element and the second out-coupling element in the pair.

The first and second waveguides as well as the common detection waveguide may be arranged in a common waveguide layer. Alternatively, the first and second waveguides may be arranged in a first waveguide layer and a second waveguide layer, respectively, forming separate layers. The common detection waveguide may further be arranged in an intermediate layer sandwiched between the first layer and the second layer.

According to another embodiment, each of the first out-coupling elements comprises a first directional coupler configured to couple the fraction of the first portion of the light signal into a first detection waveguide, and wherein each of the second out-coupling elements comprises a second directional coupler configured to couple the fraction of the second portion of light into a second detection waveguide.

Thanks to out-coupling of light into detection waveguides, the out-coupled light is further propagated in a detection waveguide allowing control of guiding of light. This may facilitate controlling of out-coupled light for forming interference signals such that light may be efficiently used for detecting the spectral information.

This may be used for first and second detection waveguides in a common waveguide layer with the first and second waveguides. This may facilitate forming a compact device.

According to an embodiment, the first detection waveguide and the second detection waveguide associated with a pair of the first out-coupling element and the second out-coupling element are connected to a combiner coupler for forming a combined signal based on interference by the fraction of the first portion of the light signal and the fraction of the second portion of the light signal.

Thus, the combined signal may be formed by combining the signals from the detection waveguides. This combined signal may further be output to a photosensitive element for detection.

The combiner coupler may for instance be formed by a directional coupler or a multi-mode interference coupler. For instance, a 2 x 2 coupler may be used for combining light input to the combiner coupler from the first detection waveguide and the second detection waveguide, respectively.

A combined signal may be output to a single photo-sensitive element. However, according to an embodiment, two combined signals may be formed and output to two photo-sensitive elements for balanced photo-detection.

According to yet another embodiment, each of the first out-coupling elements comprises a first grating coupler configured to direct the fraction of the first portion of the light signal out of the first waveguide and wherein each of the second out-coupling elements comprises a second grating coupler configured to direct the fraction of the second portion of the light signal out of the second waveguide.

The out-coupling elements may comprise diverting couplers configured to direct the fraction of the first and second portion, respectively, of the light signal out of a plane of the first waveguide and the second waveguide, respectively. This may be advantageously provided by the grating couplers.

According to an embodiment, the first grating coupler and the second grating coupler are configured to direct the fraction of the first portion of the light signal and the fraction of the second portion of the light signal onto a common photo-sensitive area.

The photo-sensitive area may be provided by a photo-sensitive element. Thus, the out-coupling elements may be configured to direct light such that an interference signal is formed on the photo-sensitive element.

According to an embodiment, the device further comprises a plurality of photo-sensitive elements, wherein each photo-sensitive element is configured to receive a combined signal formed by interference of the fraction of the first portion of the light signal and the fraction of the second portion of the light signal.

Thus, the device may include the photo-sensitive elements for detecting the combined signals. This may facilitate providing a compact device for both forming the combined signals and detecting the combined signals for spectroscopy measurements.

Each photo-sensitive element in the plurality of photo-sensitive elements may be configured to detect an intensity of light. The photo-sensitive elements may be configured to provide an electrical signal, such as a current, voltage or charge, in dependence of a received intensity of light. The photo-sensitive element may for instance be formed by a photodiode, a photo-multiplier tube (PMT), or a pixel on an image sensor, such as based on charge-coupled device (CCD) technology or on complementary metal oxide semiconductor (CMOS) technology.

The plurality of photo-sensitive elements may be arranged in an array of photo-sensitive elements, such as in a one-dimensional array or a two-dimensional array. It should be realized that the plurality of photo-sensitive elements may be regularly arranged in the array with equal distances between neighboring photo-sensitive elements in the array. Thus, an array of photo-sensitive elements may be utilized, such as an array of photo-sensitive elements arranged in an image sensor. As an alternative, the photo-sensitive elements of the plurality of photo-sensitive elements are arranged in any relation to each other and need not be regularly arranged in an array.

The plurality of photo-sensitive elements may be arranged on or in a substrate. The components of the device may be integrated on the substrate. For instance, the first and second light propagation units may be formed in one or more waveguide layers integrated on the substrate.

In other embodiments, the plurality of photo-sensitive elements may be arranged separate from the first and second light propagation units and not necessarily integrated therewith. The plurality of photo-sensitive elements may then be mounted in a fixed relation to the first and second light propagation units in order for the photo-sensitive elements to properly receive the out-coupled fractions of the first and second portions of the light signal.

According to a second aspect, there is provided a system for Fourier Transform spectroscopy, said system comprising: a polarization splitter for splitting a combined polarization signal into a first light signal having a first polarization and a second light signal having a second polarization; a first device according to the first aspect, wherein the light signal received by the first device is based on the first light signal; and a second device according to the first aspect, wherein the light signal received by the second device is based on the second light signal.

Effects and features of this second aspect are largely analogous to those described above in connection with the first aspect. Embodiments mentioned in relation to the first aspect are largely compatible with the second aspect.

It should be realized that it may be difficult to provide polarization-independent components of the device of the first aspect. For instance, the out-coupling elements may have a dependency of polarization of light.

However, the received light signal may not have any specific polarization. Thus, if only the device of the first aspect is used for receiving light, some light may be lost due to the polarization dependence of components.

Thanks to the system being configured to detect the first light signal having the first polarization and the second light signal having the second polarization, the system is able to take all or at least most of the light of the combined polarization signal into account in FT spectroscopy.

In addition, analysis of received light in dependence of polarization may also enable extracting information from the received light based on polarization. For instance, the analysis of received light using a first and a second device receiving different polarizations of light may be used for determining information relating to birefringence of a sample.

The polarization splitter may be any component which is configured to direct light in different directions based on a polarization of light. For instance, the polarization splitter may comprise a birefringent material for splitting light into two orthogonal polarization states.

The combined polarization signal may for instance be split into transverse electric (TE) polarized light forming the first light signal and a transverse magnetic (TM) polarized light forming the second light signal.

According to an embodiment, the system further comprises a polarization rotator configured to receive the second light signal and configured to provide a rotation of the second polarization for forming the light signal received by the second device.

In particular, the polarization rotator may be configured to rotate the second polarization of the second light signal to correspond to the first polarization such the light signal received by the second device has a same polarization as the light signal received by the first device. For instance, the polarization rotator may be configured to rotate a linear polarization of the second light signal by 90 degrees to correspond to a linear polarization of the first light signal.

This implies that the first and the second devices may be configured to receive light signals having identical polarization. Hence, the first and second devices may have identical components adapted to receiving a same type of polarization of light. This may facilitate manufacturing of the system, since the first and second devices may be identical.

According to a third aspect, there is provided a system for spectral domain interferometric measurement, said system comprising: a light source configured to provide illumination light; a coupler element configured to split the illumination light into a reference beam and a sample beam, wherein the system is configured to output the sample beam towards a sample for interacting with the sample beam to form a response beam carrying information of the sample; a device according to the first aspect, wherein the light signal received by the device is formed by a combination of reference beam and the response beam.

Effects and features of this third aspect are largely analogous to those described above in connection with the first and second aspects. Embodiments mentioned in relation to the first and second aspects are largely compatible with the third aspect.

This system utilizes the device according to the first aspect for a particular measurement, wherein a response beam carrying information from a sample is combined with a reference beam for extracting information from the sample. The response beam and the reference beam may be combined based on interference of the response beam and the reference beam.

The reference beam may be used for ensuring that a portion of the response beam having travelled a particular distance may be extracted. Thus, the reference beam may be formed by reflection from a mirror.

Interference between the reference beam and the response beam may provide information of position of reflecting surfaces in the sample. This may be used for optical fiber and integrated photonic component testing and material metrology.

Depth information of the sample corresponds to light traveling along different path lengths to and from the sample. Thus, information from different depths may be typically be acquired by scanning a position of the mirror in order for the reference beam to travel a path length corresponding to a particular depth.

However, thanks to the use of the device according to the first aspect for FT spectroscopy, information from different path length relations between the response beam and the reference beam may be extracted in a single measurement. This may be used for providing spectral domain interferometric measurement of interference between the reference beam and the response beam. For measurements of depth information of the sample, the system may provide depth information in a single measurement without need of moving of the mirror.

It should be realized that the system for spectral domain interferometric measurement may provide the combination of the reference beam and the response beam as the combined polarization signal received by a system according to the second aspect. Thus, the system according to the second aspect may be used for analysis of the sample based on the information carried in the combination of the reference beam and the response beam.

According to an embodiment, the system is configured to perform a reference measurement, wherein the light signal received by the device, during the calibration measurement, is formed by the reference beam.

Thanks to performing of the reference measurement, impact on the combined light signal of the reference beam and the response beam due to the light travelling in a reference arm may be removed. This facilitates extracting the depth information from the sample.

According to an embodiment, the system is configured to output the sample beam towards biological tissue, such as a retina, for performing Optical Coherence Tomography (OCT).

In OCT, depth information from a sample, such as a retina, is to be acquired. This may conventionally be performed by using a movable mirror for providing different path lengths of the reference beam. However, thanks to the use of the system according to the third aspect, moving of the mirror in the reference arm is not needed and the system may be configured to extract depth information of the sample with a stationary mirror.

Thus, the system is particularly suited for performing OCT measurements. However, it should be realized that the system may be used in various other applications as well.

According to a fourth aspect, there is provided a method for guiding a light signal for Fourier Transform spectroscopy, said method comprising: splitting a light signal into a first portion and a second portion; propagating the first portion of the light signal in a first waveguide passing a sequence of first out-coupling elements; coupling, by first out-coupling elements in the sequence, a fraction of the first portion of the light signal out of the first waveguide with a remaining fraction of the first portion of the light signal continuing propagation along the first waveguide; propagating the second portion of the light signal in a second waveguide passing a sequence of second out-coupling elements; coupling, by second out-coupling elements in the sequence, a fraction of the second portion of the light signal out of the second waveguide with a remaining fraction of the second portion of the light signal continuing propagation along the second waveguide; wherein an optical path length along the second waveguide between sequential second out-coupling elements is different than an optical path length along the first waveguide between corresponding sequential first out-coupling elements, wherein the first out-coupling elements and second out-coupling elements form a sequence of pairs, each comprising one second out-coupling element and one corresponding first out-coupling element, wherein a difference in the optical path length propagated by the fraction of the second portion of the light signal out-coupled by the second out-coupling element compared to the optical path length propagated by the fraction of the first portion of the light signal out-coupled by the corresponding first out-coupling element is configured to be different along the sequence of pairs.

Effects and features of this fourth aspect are largely analogous to those described above in connection with the first, second, and third aspects. Embodiments mentioned in relation to the first, second, and third aspects are largely compatible with the fourth aspect.

Thanks to the method, multiple out-coupling elements are arranged in each of a first light propagating unit and a second light propagating unit. This implies that a difference in optical path length provided for a first pair of light-coupling elements may also be utilized by following pairs of light-coupling elements. Hence, the difference in optical path length for a particular pair of out-coupling elements may be formed by a sum of differences in the sequence of pairs such that the method may provide output for forming a plurality of interference signals in a compact manner.

Further, thanks to the out-coupling of a fraction of the portion of the light signal and a remaining fraction being passed for continuing propagation along the waveguide, out-coupling elements in the sequence may simultaneously out-couple light for detection. This implies that the interference corresponding to different path length relations may be simultaneously provided for detection. This implies that interference signals for allowing detection of FT spectroscopy may be simultaneously formed and hence simultaneously detected. Thus, the method facilitates acquiring information for FT spectroscopy in a fast manner.

It should be realized that the method of the fourth aspect is directed to the out-coupling of light for forming interference signals allowing signals for FT spectroscopy to be detected. However, the detection may not necessarily be performed within the device of the first aspect and the method is therefore defined as not necessarily performing actual detection, since this may be performed by a different device.

The method therefore relates to guiding of a light signal, wherein the light signal is affected such that FT spectroscopy may be performed. The method of guiding of the light signal may thus be performed in FT spectroscopy.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a schematic view of graphs illustrating function of a Fourier Transform Spatial Heterodyne Spectrometer.

Fig. 2a is a schematic top view of a device for Fourier Transform spectroscopy according to a first embodiment.

Fig. 2b is a schematic cross-sectional view along line B-B of the device in Fig. 2a.

Fig. 2c is a schematic cross-sectional view along line C-C of the device in Fig. 2a.

Fig. 3a-b are schematic top views of a device for Fourier Transform spectroscopy according to a second embodiment.

Figs 4a-c are schematic top views with inserted cross-sectional views of a device for Fourier Transform spectroscopy according to a third embodiment.

Fig. 5 is a schematic view of a system for Fourier Transform spectroscopy according to an embodiment.

Fig. 6 is a schematic view of a system for spectral domain interferometric measurement according to an embodiment which may be used in Optical Coherence Tomography.

Fig. 7 is a schematic view of a method for performing measurements by the system of Fig. 6.

Fig. 8 is a schematic view of a method for performing calibration by the system of Fig. 6.

Fig. 9 is a flow chart of a method performed by a device according to any of the first to third embodiments.

Detailed description

A Fourier Transform Spatial Heterodyne Spectrometer (FT-SHS) is a tool for analysis of spectral content of a light signal. The FT-SHS may for instance be used for analyzing samples in various sensing applications, particularly in spectroscopy.

An operating principle of FT-SHS will now be generally described. The FT-SHS may comprise multiple (N) asymmetric Mach-Zehnder interferometers (MZIs), where two arms of each MZI have different optical delays. Thus, a light signal is split and allowed to propagate through each of the two arms of the MZI. Thereafter, the split light signals are combined again to form an interference which is dependent on the difference of the optical delays provided by the two arms. The intensities of the combined signals are measured by respective photo-sensitive elements, and the intensities depend on the phase difference of the combined signals due to the difference of the optical delays. Different unbalanced optical delays are provided in the MZIs. These unbalanced optical delays may all be a multiple of a smallest delay $\Delta L$, such that the unbalanced optical delays provided in the MZIs may be $\Delta L$, $2\Delta L$, ... , $N\Delta L$.

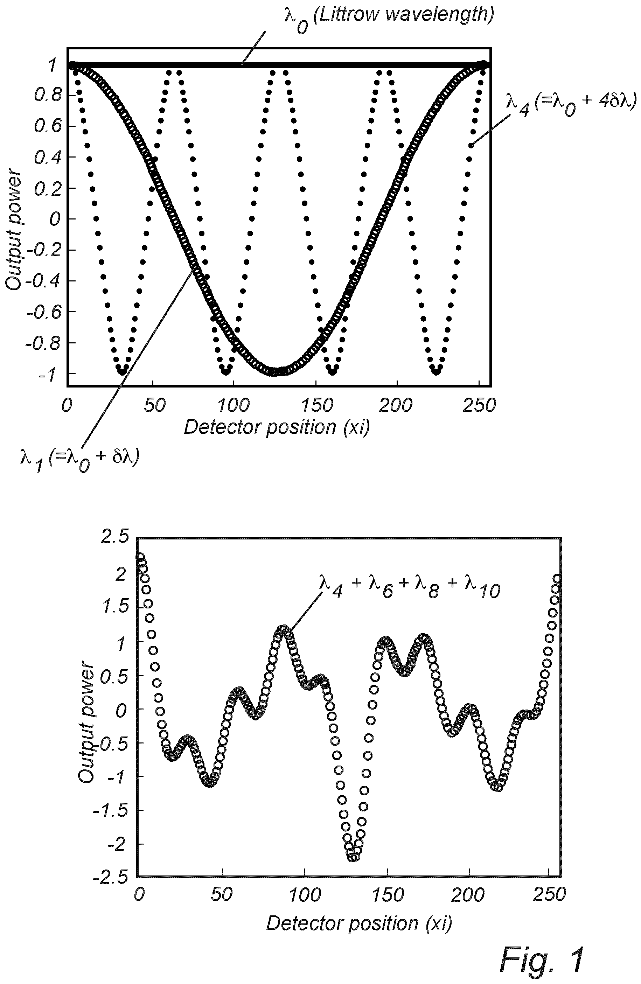

In Fig. 1, detected light intensities associated with the $N$ MZIs is illustrated in a left-hand graph. The difference in optical delay for the MZIs increases along an x-axis of the left-hand graph. At a specific wavelength, $\lambda_0$, which is called the Littrow wavelength, the phase delays in different MZIs are integer multiples of $2\pi$, producing a constant output.

When the frequency of the light signal increases by $\delta\lambda$ (i.e., to $\lambda_0 + \delta\lambda$), the output signal displays one spatial fringe. This wavelength difference $\delta\lambda$ corresponds to a spectral resolution of the FT-SHS. Correspondingly, for a light signal with a wavelength of $\lambda_0 + 4\delta\lambda$, four fringes are produced.

Fig. 1 further illustrates in a right-hand graph, a polychromatic light signal, comprised of multiple discrete wavelengths, which produces a specific fringe pattern representing a superposition of multiple fringes of respective wavelengths. The determined light intensities may then be converted using the Fourier transform (FT) to retrieve the signal spectrum. Such signal spectrum may then be used, for example, to determine properties of a sample that generated the light signal input to the FT-SHS.

The spectral resolution, $\delta\lambda$, of the spectrometer is related to a maximum unbalanced path delay $\Delta L_{max} = N\Delta L$. The spectral resolution can be calculated as follows: $\delta\lambda = \dfrac{\lambda_0^2}{\Delta L_{max} \cdot n_{eff}}$, where $n_{eff}$ is an effective refractive index of a waveguide used in the MZIs. Thus, the spectral resolution may be improved by simply increasing $\Delta L_{max}$.

Based on Fourier sampling theorem, the number of MZIs ($N$) is obtained as follows:

$$N = \frac{2\Delta\lambda}{\delta\lambda},$$

where $\Delta\lambda$ is a wavelength range of the measurement. Hence, a large wavelength range can be achieved by increasing $N$ for a given $\Delta L_{max}$. As such, FT-SHS has low fabrication difficulties in achieving small wavelength resolution and a large range, representing good scalability. However, FT-SHS requires a relatively large area especially to achieve small resolution and large bandwidth as many MZIs are required. This and other problems are addressed by aspects of the present description which will now be described.

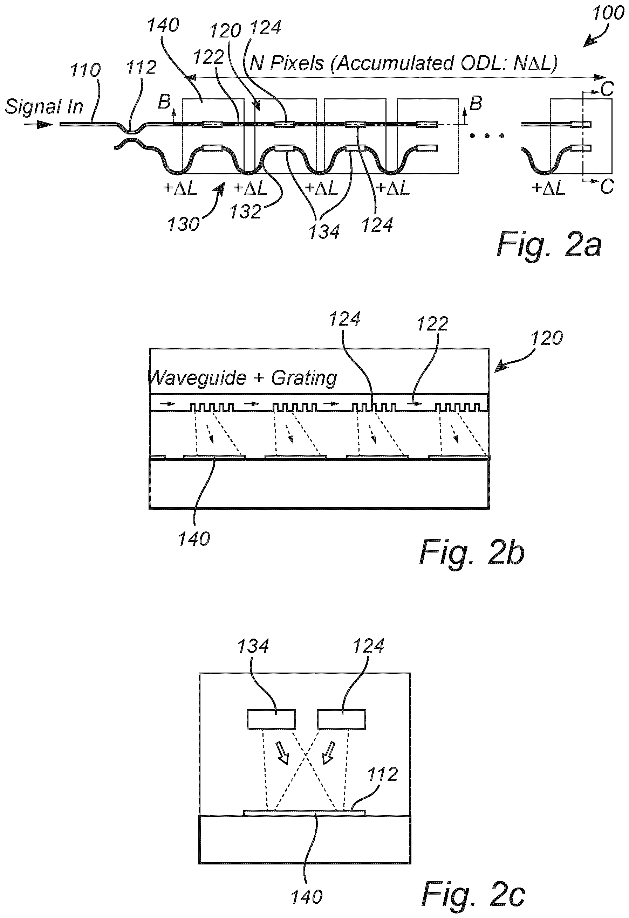

Referring now to Figs 2a-c, a device 100 for Fourier Transform spectroscopy according to a first embodiment will be described. The device 100 is configured to form combined signals for different optical delays as described above. However, the device 100 is configured to form these combined signals in a compact manner with low loss of light.

The device 100 comprises an input waveguide 110 for receiving a light signal. The light signal may be any signal for which spectral information is to be determined, such as a signal from a sample representing for instance transmission of light through the sample or reflection of light by the sample or any other interaction of light by the sample.

The input waveguide 100 may be configured to extend in a plane for transporting the light signal. The input waveguide 110 may comprise a grating coupler for receiving the light signal. This may be used for receiving the light signal from outside the plane in which the input waveguide 110 extends. However, it should be realized that the input waveguide 110 may receive the light signal in another suitable manner, such as through an edge of the waveguide 110.

The input waveguide 110 is configured to transport the light signal to a splitter 112. The splitter 112 is connected to the input waveguide 110. The splitter 112 is configured to split the light signal into a first portion and a second portion. The splitter 112 may thus direct the first portion to a first light propagating unit 120 connected to the splitter 112 and direct the second portion to a second light propagating unit 130 connected to the splitter.

The splitter 112 may be implemented by any component configured to split the light signal into multiple portions. For instance, the splitter 112 may be implemented by a multi-mode interference (MMI) coupler, such as a $1 \times 2$ MMI coupler. According to an alternative, the splitter 112 may be implemented by a directional coupler.

The first light propagating unit 120 and the second light propagating unit 130 are configured to provide propagation of the first and second portions of the light signal, respectively, such that the first and second portions of the light signal propagate through different optical path lengths.

Each light propagating unit comprises a waveguide transporting the portion of the light signal through a sequence of out-coupling elements. Out-coupling elements in the sequence couple a fraction of the portion of the light signal transported in the waveguide out of the waveguide, whereas a remaining fraction of the portion of the light continues propagation in the waveguide towards a following out-coupling element in the sequence.

Corresponding out-coupling elements in the first and the second light propagation units 120, 130 form a pair of out-coupling elements. The fractions of light out-coupled from the out-coupling elements in a pair is provided to be combined for forming an interference signal which may be detected. The optical path lengths between corresponding sequential out-coupling elements in the first and second light propagation units 120, 130 differ. Thus, between each pair of out-coupling elements, a difference in optical path lengths travelled by the first portion of the light signal and by the second portion of the light signal is added. This implies that a later pair of out-coupling elements may utilize the optical path length difference provided by an earlier pair of out-coupling elements and there is only a need of providing an additional difference in optical path length. This implies that a plurality of different optical path lengths may be provided in a very compact manner.

In other words, the first light propagating unit 120 is configured to receive the first portion of the light signal. The first light propagating unit 120 may receive the first portion of the light signal in a first waveguide 122 connected to the splitter 112. The first waveguide 122 is configured to propagate the first portion of the light signal to a sequence of first out-coupling elements 124. The first out-coupling elements 124 are spaced apart along an extension of the first waveguide 122. The first out-coupling elements 124 in the sequence are configured to couple a fraction of the first portion of the light signal out of the first waveguide 122 with a remaining fraction of the first portion of the light signal continuing propagation along the first waveguide 122 towards a following out-coupling element 124 in the sequence.

The second light propagating unit 130 is configured to receive the second portion of the light signal. The second light propagating unit 130 may receive the second portion of the light signal in a second waveguide 132 connected to the splitter 112. The second waveguide 132 is configured to propagate the second portion of the light signal to a sequence of second out-coupling elements 134. The second out-coupling elements 134 are spaced apart along an extension of the second waveguide 132. The second out-coupling elements 134 in the sequence are configured to couple a fraction of the second portion of the light signal out of the second waveguide 132 with a remaining fraction of the second portion of the light signal continuing propagation along the second waveguide 132 towards a following out-coupling element 134 in the sequence.

An optical path length along the second waveguide 132 between sequential second out-coupling elements 134 is different than an optical path length along the first waveguide 122 between corresponding sequential first out-coupling elements 124. A difference in the optical path length propagated by the fraction of the second portion of the light signal out-coupled by the second out-coupling element 134 compared to the optical path length propagated by the fraction of the first portion of the light signal out-coupled by the corresponding first out-coupling element 124 is configured to be different along the sequence of pairs.

Thus, the pairs of out-coupling elements may provide output of light signals for providing the different optical path length differences between the first and second portions of the light signal such that information for FT spectroscopy may be detected.

According to an embodiment, the optical path length along the second waveguide 132 between sequential second out-coupling elements 134 is always larger than the optical path length along the first waveguide 122 between corresponding sequential first out-coupling elements 124, wherein the difference in the optical path length propagated by the fraction of the second portion of the light signal out-coupled by the second out-coupling element 134 compared to the optical path length propagated by the fraction of the first portion of the light signal out-coupled by the corresponding first out-coupling element 124 is configured to increase along the sequence of pairs. This implies that path length differences are continuously added along the sequence of pairs for providing a compact arrangement of the light propagation units.

Further, since the out-coupling elements 124, 134 output a fraction of the first portion of the light signal and the second portion of the light signal, respectively, fractions of light may be combined simultaneously from the out-coupling elements 124, 134. This enables simultaneous detection of the information for Fourier spectroscopy while providing a compact arrangement.

The differences in optical path lengths may configured to increase along the sequence of pairs by an equal added path length between sequential pairs. The equal added path length may be a smallest delay $\Delta L$ to be detected, such that the differences in optical path length delays defined by the pairs of out-coupling elements form multiples of the smallest delay. Thus, the unbalanced optical delays provided by the output from the pairs of out-coupling elements may be $\Delta L$, $2\Delta L$, ... , $N\Delta L$.

As shown in Figs 2a-c, the waveguides 122, 132 of the first and second light propagating units 120, 130 may be arranged in a common waveguide layer. However, it should be realized that the waveguides 122, 132, may alternatively be provided in different layers.

At least one of the waveguides 122, 132 may be configured to extend along a non-straight path for defining an optical path length delay. In Fig. 2a, the second waveguide 132 is configured to follow a curved path between sequential out-coupling elements 134, while the first waveguide 122 follows a straight path between sequential out-coupling elements 124. This implies that an optical path length difference is provided between the paths travelled by the first portion of the light signal and the second portion of the light signal. The out-coupling elements 124, 134 of a pair may be arranged close to each other. Thus, the second waveguide 132, between two out-coupling elements 134 may be configured to extend away from the first waveguide 122 and then back towards the first waveguide 122 for providing an additional optical path length while providing the second out-coupling elements 134 in close relation to corresponding first out-coupling elements 124.

However, it should be realized that optical path length differences may be achieved in other manners, such as by using different refractive indices of the waveguides 122, 132. This may be achieved by having different materials of the waveguides 122, 132 or by heating one of the waveguides 122, 132 for changing the refractive index.

The optical path length differences may be provided by the design of the device 100. Thus, no active control may be needed for allowing detection of the information for Fourier spectroscopy. However, as mentioned above, active control may be used for defining the optical path length difference. In addition, active control may be provided for fine-tuning an optical path length difference, e.g., for compensating for manufacturing errors. Thus, a thermo-optic or electro-optic element configured to change refractive index of the waveguide 122, 132 may be associated with at least one of the waveguides 122, 132 for controlling the optical path length differences. For instance, throughout the sequence of second out-coupling elements 134, a phase shifter is associated with the waveguide 132 between each sequential out-coupling elements 134.

Fig. 2b shows a cross-section taken along line B-B in Fig. 2a, illustrating the first waveguide 122 of the first light propagating unit 120 extending along the sequence of first out-coupling elements 124. The first out-coupling elements 124 may be arranged in the first waveguide 122 to divert a fraction of light out of the waveguide 122. This may be achieved by each out-coupling element 124 comprising a grating coupler. The grating coupler may thus be configured to direct the fraction of the first portion of the light signal out of the first waveguide 122 while a remaining fraction continues propagation in the first waveguide 122.

Similarly, the second out-coupling elements 134 may be arranged in the first waveguide 132 to divert a fraction of light out of the waveguide 132. This may be achieved by each out-coupling element 134 comprising a grating coupler. The grating coupler may thus be configured to direct the fraction of the second portion of the light signal out of the second waveguide 132 while a remaining fraction continues propagation in the second waveguide 132.

Fig. 2c shows a cross-section taken along line C-C in Fig. 2a, illustrating a pair of out-coupling elements in the first waveguide 122 and the second waveguide 132. The out-coupling elements may be configured to direct out-coupled light towards a common photo-sensitive area 142 of a photo-sensitive device 140.

The first out-coupling elements 124 in the sequence of first out-coupling elements 124 are configured to couple out an increasing fraction of the light signal from the first waveguide 122 in the sequence. Also, the second out-coupling elements 134 in the sequence of second out-coupling elements 134 are configured to couple out an increasing fraction of the light signal from the second waveguide 132 in the sequence.

Thus, the out-coupling elements 124, 134 in the sequences may have different settings such that the ratio of light being out-coupled by the out-coupling elements 124, 134 increases along the sequence. This implies that the optical powers output by each of the out-coupling elements 124, 134 may be identical or at least substantially identical.

The device 100 may be formed as photonic integrated circuit (PIC). It should be realized that the first and second light propagation units 120, 130 may be formed in a PIC. In addition, the input waveguide 110 and the splitter 112 may also be formed in the same PIC. This may be a suitable manner for providing a very compact device 100.

The device 100 may not necessarily include photo-sensitive elements for detecting combined signals from the pairs of out-coupling elements. For instance, the photo-sensitive elements may be defined on a separate substrate which may be appropriately mounted in relation to the first and second light propagation units 120, 130.

However, the device 100 may comprise a plurality of photo-sensitive elements 140. As shown in Figs 2a-c, the photo-sensitive elements 140 may be integrated with the first and second light propagation units 120, 130. The waveguides 122, 132 may be arranged in a waveguide layer integrated with a substrate in which the photo-sensitive elements 140 are formed. The photo-sensitive elements 140 may for instance be provided as pixels in a complementary metal-oxide-semiconductor (CMOS) image sensor.

Each pair of out-coupling elements is associated with a respective photo-sensitive element 140 for detecting the intensity of light out-coupled by the pair of out-coupling elements.

The out-coupled fractions of the first portion of the light signal and the second portion of the light signal may be incident on the photo-sensitive element 140 for providing interference between the fractions of light.

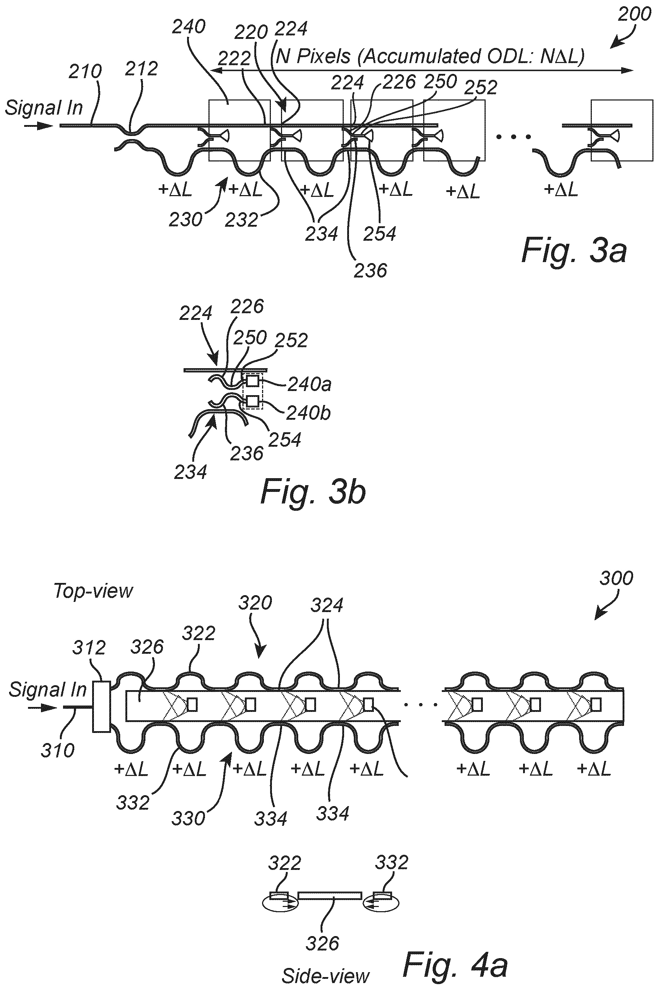

Referring now to Fig. 3a, a device 200 according to a second embodiment will be described. The device 200 shares features with the device 100 of the first embodiment and, for brevity, only differences between the embodiments will be described below in detail.

The device 200 comprises an input waveguide 210 and a splitter 212 for splitting the received light signal into a first portion received by a first light propagation unit 220 and a second portion received by a second light propagating unit 230.

The first light propagating unit 220 comprises a first waveguide 222 for transporting the first portion of the light signal to a plurality of first out-coupling elements 224 arranged in a sequence. The second light propagating unit 230 comprises a second waveguide 232 for transporting the second portion of the light signal to a plurality of second out-coupling elements 234 arranged in a sequence. The optical path length along the second waveguide 232 between sequential second out-coupling elements 234 is different than the optical path length along the first waveguide 222 between corresponding sequential first out-coupling elements 224.

The out-coupling elements 224, 234 in the device 200 according to the second embodiment are implemented in a different manner to the device 100 according to the first embodiment. A first out-coupling element 224 comprises a first directional coupler configured to couple the fraction of the first portion of the light signal into a first detection waveguide 226 while a remaining fraction is allowed to continue propagation in the first waveguide 222. A second out-coupling elements 234 comprises a second directional coupler configured to couple the fraction of the second portion of light into a second detection waveguide 236.

The first detection waveguide 226 and the second detection waveguide 236 associated with a pair of the first out-coupling element 224 and the second out-coupling element 234 are connected to a combiner coupler 250 for forming a combined signal based on interference by the fraction of the first portion of the light signal and the fraction of the second portion of the light signal propagating in the first detection waveguide 226 and the second detection waveguide 236, respectively.

The combiner coupler 250 may be implemented by any component that may combine the light propagating in the first and second detection waveguides 226, 236. The combiner coupler 250 may for instance be implemented by another directional coupler or by a MMI coupler, such as a 2 x 2 MMI coupler. Thus, the fractions of the first portion of the light signal and the second portion of the light signal may be combined based on interference in a waveguide 252. Thus, the combined signal is well-controlled. This facilitates ensuring that the fractions of light properly interfere and may be detected with low loss of light.

A coupling efficiency of the first directional couplers in the sequence of first out-coupling elements 224 is configured to increase in the sequence. Also, a coupling efficiency of the second directional couplers in the sequence of second out-coupling elements 234 is configured to increase in the sequence.

This implies that the optical powers output by each of the out-coupling elements 224, 234 may be identical or at least substantially identical.

The pair of out-coupling elements may further be associated with a diverting element 254 for diverting the combined light signal towards a photo-sensitive element 240.

Like the device 100 of the first embodiment, the device 200 of the second embodiment may be formed by a PIC. The photo-sensitive elements 240 may be external to the device 200 or may form part of the device 200, e.g., by being part of an integrated device 200.

In Fig. 3b, a variant of the device 200 of the second embodiment is shown. In Fig. 3b, a single pair of out-coupling elements 224, 234 is shown together with a detector for detecting the combined light signal. It should be realized that each of the pairs of out-coupling elements and each photo-sensitive element in Fig. 3a may instead be implemented as shown in Fig. 3b.

Thus, the pair of out-coupling elements 224, 234 may be configured to couple light into a first detection waveguide 226 and a second detection waveguide 236, respectively. The first detection waveguide 226 and the second detection waveguide 236 are further connected to the combiner coupler 250 for forming a combined signal based on interference.

The combiner coupler 250 may for instance be a 2 x 2 MMI coupler or directional coupler and may be configured to output two combined signals to two waveguides 252, 256. Each of the waveguides 252, 256 connected to the combiner coupler 250 may further be connected to a respective photo-sensitive element 240a, 240b. Thus, two combined signals may be output to two photo-sensitive elements 240a, 240b for balanced photo-detection.

Referring now to Figs 4a-c, a device 300 according to a third embodiment will be described. The device 300 shares features with the devices 100, 200 of the first and second embodiments and, for brevity, only differences between the embodiments will be described below in detail.

The device 300 comprises an input waveguide 310 and a splitter 312 for splitting the received light signal into a first portion received by a first light propagation unit 320 and a second portion received by a second light propagating unit 330.

The first light propagating unit 320 comprises a first waveguide 322 for transporting the first portion of the light signal to a plurality of first out-coupling elements 324 arranged in a sequence. The second light propagating unit 330 comprises a second waveguide 332 for transporting the second portion of the light signal to a plurality of second out-coupling elements 334 arranged in a sequence. The optical path length along the second waveguide 332 between sequential second out-coupling elements 334 is different than the optical path length along the first waveguide 322 between corresponding sequential first out-coupling elements 324.

The out-coupling elements 324, 334 in the device 300 according to the second embodiment are implemented in a different manner to the devices 100, 200 according to the first and second embodiments. The first and second waveguides 322, 332 may be arranged in relation to a common detection waveguide 326. At the out-coupling elements 324, 334, the first and second waveguides 322, 332 may be arranged close to the common detection waveguide 326, whereas between the out-coupling elements 322, 332, the out-coupling elements 324, 334 are not arranged close to the common detection waveguide 326 for causing any out-coupling of light. Thus, a sequence of out-coupling elements 324, 334 may be formed by arranging the first and second waveguides 322, 332, respectively, close to the common detection waveguide 326.

The arranging the first and second waveguides 322, 332, respectively, close to the common detection waveguide 326 implies that a fraction of the first portion of the light signal and a fraction of the second portion of the light signal may be coupled into the common detection waveguide 326 based on an evanescent field.

Thus, out-coupling of the fraction of the first portion of the light signal and out-coupling of the fraction of the second portion of the light signal by each pair of first and second out-coupling elements is configured to be received directly in the common detection waveguide 326. Thus, the fractions of the first portion of the light signal and the second portion of the light signal may be combined based on interference directly in the common detection waveguide 326. This provides for a very compact device 300.

As shown in Fig. 4a, the common detection waveguide 326 may extend continuously along the sequence of pairs of out-coupling elements. The fractions of the first portion of the light signal and the second portion of the light signal may be coupled into the common detection waveguide 326 at the respective locations where the first waveguide 322 and the second waveguide 332 are arranged in close relation to the common detection waveguide 326. The combined signal may then be out-coupled to a photo-sensitive element 340 for detection. The photo-sensitive element 340 may be arranged in a layer in which the common detection waveguide 326 is arranged.

A coupling efficiency of the first out-coupling elements in the sequence of first out-coupling elements 324 is configured to increase in the sequence. Also, a coupling efficiency of the second out-coupling elements in the sequence of second out-coupling elements 334 is configured to increase in the sequence.

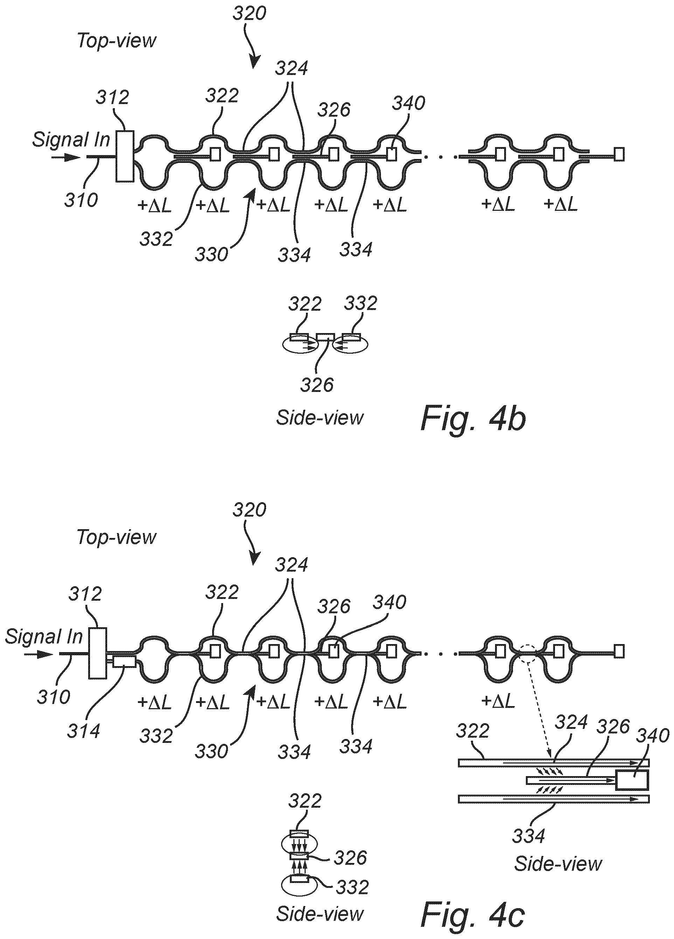

As shown in Fig. 4b, each pair of out-coupling elements may be associated with a separate common detection waveguide 326. The first and second waveguides 322, 332, respectively, are arranged close to the common detection waveguide 326 at the out-coupling elements such that a fraction of the first portion of the light signal and a fraction of the second portion of the light signal may be coupled into the common detection waveguide 326 based on an evanescent field.

In Fig. 4b, the first and second waveguides 322, 332 are arranged in a common waveguide layer and the common detection waveguide 326 is also arranged in the common waveguide layer. The first and second waveguides 322, 332 are arranged at opposite sides of the common detection waveguide 326 in the common waveguide layer for coupling light into the common detection waveguide 326. The common detection waveguide 326 may guide light to a photo-sensitive element 340 for detection.

In Fig. 4c, like in Fig. 4b, each pair of out-coupling elements is associated with a separate common detection waveguide 326. The first and second waveguides 322, 332, respectively, are arranged close to the common detection waveguide 326 at the out-coupling elements such that a fraction of the first portion of the light signal and a fraction of the second portion of the light signal may be coupled into the common detection waveguide 326 based on an evanescent field.

In Fig. 4c, the first and second waveguides 322, 332 are arranged in first and second waveguide layers, respectively, and the common detection waveguide 326 is arranged in a waveguide layer arranged between the first and second waveguide layers. The device 300 may comprise a coupling element 314 for coupling the second portion of the light signal from the first waveguide layer to the second waveguide layer such that the second portion of the light signal is brought to the second waveguide layer to be propagated therein by the second light propagating unit 330. The first and second waveguides 322, 332 are thus arranged at opposite sides of the common detection waveguide 326 with respect to a plane in which the common detection waveguide 326 extends. The common detection waveguide 326 may guide light to a photo-sensitive element 340 for detection.

The devices 100, 200, 300 of the first, second, and third embodiments provide a compact size of the FT-SHS. Additionally, waveguide sharing is leveraged to minimize relative phase errors. The devices 100, 200, 300 of the first, second, and third embodiments can achieve high resolution by simply increasing the unbalanced optical delay ($N\Delta L$).

The devices 100, 200, 300 of the first, second, and third embodiments are applicable to various fields. For instance, the devices 100, 200, 300 of the first, second, and third embodiments may be used in any type of spectroscopy, such as Raman spectroscopy.

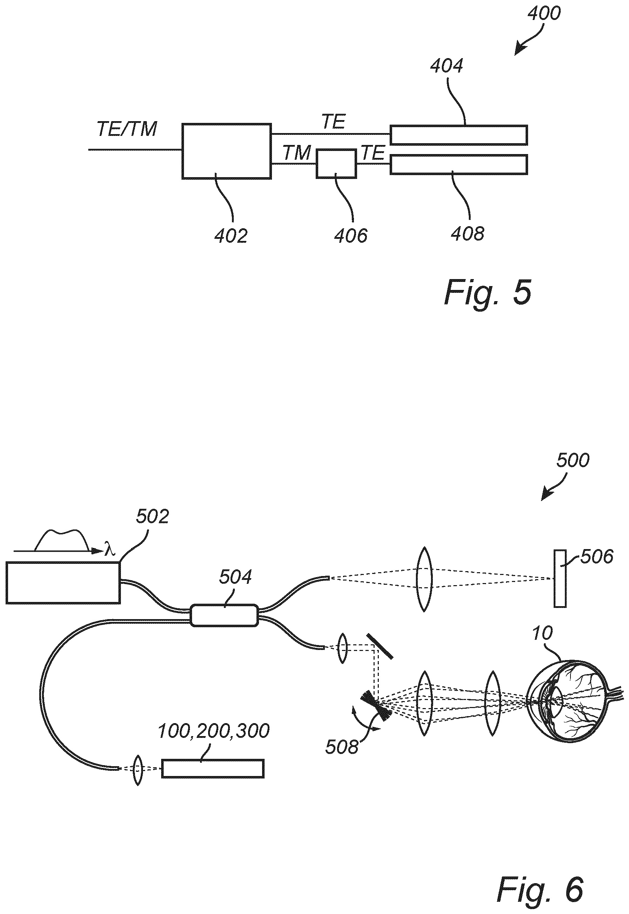

Referring now to Fig. 5, a system 400 for Fourier Transform spectroscopy will be described. It should be realized that optical components in the devices 100, 200, 300 of the first, second, and third embodiments may not be polarization-independent, or it may be difficult to design the components to be polarization-independent.

However, the received light signal may be a combined polarization signal such that it is not limited to a single polarization. Thus, if the devices 100, 200, 300 have polarization dependent components, a portion of the received light signal may be lost.

According to the system 400, the received light signal is first received by a polarization splitter 402 for splitting the combined polarization signal into a first light signal having a first polarization and a second light signal having a second polarization. This implies that each polarization may be detected separately such that light is not lost. For instance, the first light signal may be transverse electric (TE) polarized light and the second light signal may be transverse magnetic (TM) polarized light.

The system 400 further comprises a first device 404, which may be implemented as described for any of the devices 100, 200, 300 of the first, second, and third embodiments. The first device 404 may be configured to receive a signal based on the first light signal and may be configured to detect spectral information of the first light signal as described above. For instance, the first device 404 may receive TE polarized light.

The system 400 further comprises a second device 408, which may be implemented as described for any of the devices 100, 200, 300 of the first, second, and third embodiments. The second device 408 may be configured to receive a signal based on the second light signal and may be configured to detect spectral information of the second light signal as described above.

The second device 408 may receive TM polarized light. However, as shown in Fig. 5, the system 400 may further comprise a polarization rotator 406, which may receive the second light signal from the polarization splitter 402. Thus, the polarization rotator 406 is configured to provide a rotation of the second polarization before the light is input to the second device 408. The polarization rotator 406 may for instance convert the TM polarized light to TE polarized light. Thus, both the first and the second devices 404, 408 may receive TE polarized light. This implies that the first and the second devices 404, 408 may be identical.

Referring now to Fig. 6, a system 500 for spectral domain interferometric measurement will be described. The system 500 is configured to detect a combination of a reference beam and a response beam from a sample. Such a system 500 may for instance be used in Optical Coherence Tomography (OCT) but it should be realized that the system 500 may be used in other applications as well.

In particular, in the discussion below, depth information of a sample is determined corresponding to different path lengths of a response beam from the sample. However, it should be realized that the spectral domain interferometric measurement may instead be used for detecting a combination of a reference beam and a response beam, wherein the response beam is a reflection from a reflecting surface in the sample. This may be used for determining a position of the reflecting surface in the sample, which may be used for e.g., optical fiber and integrated photonic component testing and material metrology.

The system 500 may be configured to implement Fourier Domain OCT (FD-OCT). The system 500 may comprise a light source 502 configured to provide illumination light. The light source 502 may for example be a broadband light source providing light in a broad range of wavelengths.

The light source 502 may be connected to a coupler element 504. The coupler element 504 may be configured to split the illumination light into a reference beam and a sample beam, which may be follow a reference path and a sample path, respectively. The coupler element 504 may for instance be implemented as a 2 x 2 MMI coupler. The coupler element 504 may be connected to waveguides forming inputs and outputs to the coupler element 504.

The reference beam and the sample beam, respectively, may be configured to propagate in free space, such that the reference beam and the sample beam are output from waveguides for propagation in free space. The reference beam may be output towards a reference mirror 506 for traveling an optical path along the reference path to the reference mirror 506 and back towards the coupler element 504. Further optical components, such as a lens may be present in the reference path for shaping the reference beam.

The sample beam may be output towards a scanning mirror 508, which may be adjustable for controlling a direction of the sample beam. The scanning mirror 508 may be configured to direct the sample beam towards the sample, such as a retina of an eye 10. The sample beam may be reflected by the sample to form a response beam carrying information of the sample. The response beam is reflected back through the sample path towards the coupler element 504. Further optical components, such as lenses and mirrors may be present in the sample path for shaping and directing the sample beam and the response beam.

Thus, the coupler element 504 may be configured to receive the reference beam reflected by the reference mirror 506 and the response beam from the sample. The coupler element 504 may be configured to form a combination of the reference beam and the response beam based on interference of the reference beam with the response beam. The coupler element 504 may further be configured to output the combination of the reference beam and the response beam to a device for determining information in the response beam.

The output of the combination of the reference beam and the response beam from the coupler element 504 may thus be connected to a device 100, 200, 300 according to any of the above-described embodiments, which may optionally be incorporated in a system 400 as described above.

Thus, the device 100, 200, 300 according to any of the above-described embodiments may be used for extracting spectral information from the received signal. The device 100, 200, 300 is configured to form a plurality of signals based on unbalanced optical delays providing a representation of reflectance along retina layers as a function of the depth.

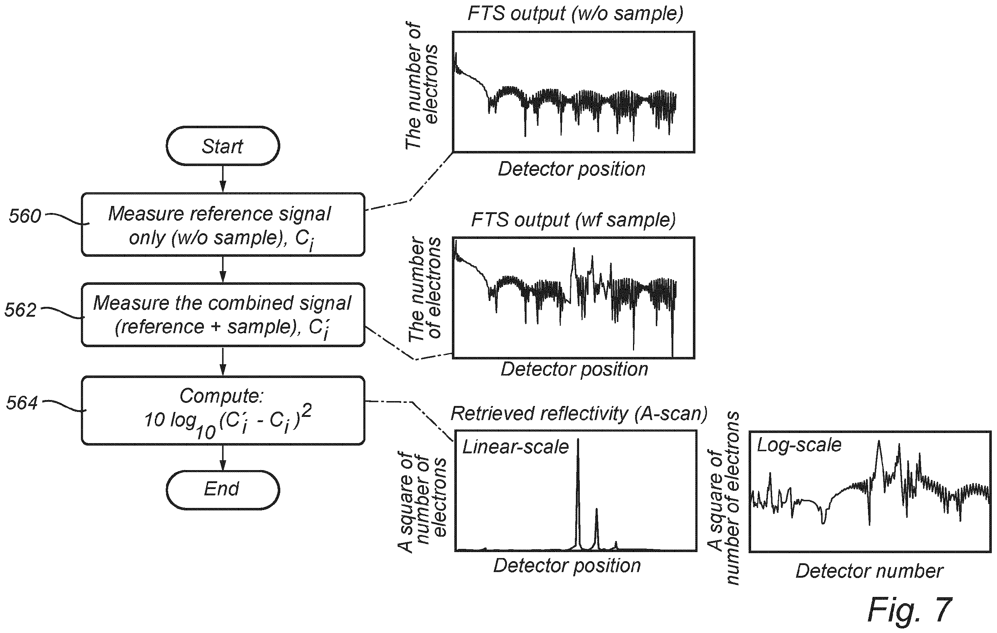

Fig. 7 illustrates a procedure to measure the depth information of the sample, such as a retina, using the device 100, 200, 300. This is illustrated in form of a flow chart representing a method that may be performed by the system 500.

First, the system 500 is configured to perform a reference measurement, wherein the light signal received by the device 100, 200, 300, during the reference measurement, is formed by the reference beam. Thus, a reference signal without the sample, $C_i$, is measured and saved 560. A reference measurement is illustrated in the graph associated with step 560.

Then, a measurement with the sample is performed 562, wherein the device 100, 200, 300 is configured to receive and measure a combination of reference and response signals, $C_i'$, is measured. This measurement is illustrated in the graph associated with step 562.

By subtracting 564 the reference signal $C_i$ from the combined signal $C_i'$, a relative reflection at a specific point of the sample, such as the retina, is obtained. A result of this subtraction in linear scale is illustrated in the left-hand graph associated with step 564. The value from the subtraction is then converted to a log scale to create image data for display as illustrated in the right-hand graph associated with step 564.

Depth information of the sample corresponds to light traveling along different path lengths to and from the sample. The device 100, 200, 300 detect signals corresponding to different path length relations, allowing information from different path length relations between the response beam and the reference beam to be extracted in a single measurement. However, there is a common mode influence originating from the reference beam, which is removed by the subtracting of the reference signal $C_i$ from the combined signal $C_i'$.

The method described above produces reflectivity vs. depth information at a single point of the sample. This can be repeated for different points (enabled by the transverse scanning mirror 508 in Fig. 6), allowing generation of a 2D or a 3D image. For 3D imaging, the transverse scanning mirror 508 needs to be scanned in both x- and y-directions, which takes relatively long time. However, thanks to the device 100, 200, 300 enabling simultaneous detection of the plurality of signals based on unbalanced optical delays, there is no need of line-scanning in the device 100, 200, 300. A camera in a spectrometer may typically have a line scanning speed (e.g., 1 ms per scan), making it difficult to create a 3D image of a retina due to eye motion artifacts. However, the device 100, 200, 300 according to any of the above embodiments does not require any line scanning, reducing an acquisition time for 2D and 3D image datasets of the sample.

A resolution and range of the OCT data along the sample, such as the retina, depend on design of the device 100, 200, 300. The resolution $\delta z$ and measurement range $\Delta z$ are given by:

$$\delta z = \Delta L \cdot \frac{n_{eff}}{n_s},$$

$$\Delta z = \delta z \cdot \frac{N}{2},$$

where $\Delta L$ is the optical path length difference between sequential pairs in the device 100, 200, 300, $n_{eff}$ is an effective refractive index of a waveguide in the device 100, 200, 300, $n_s$ is the refractive index of the sample (for instance, the eye), and $N$ is a number of pairs of out-coupling elements in the device 100, 200, 300.

The device 100, 200, 300 has reduced phase error compared to conventional FT-SHS based on MZIs. However, the device 100, 200, 300 may have some residual phase errors leading to signal distortion.

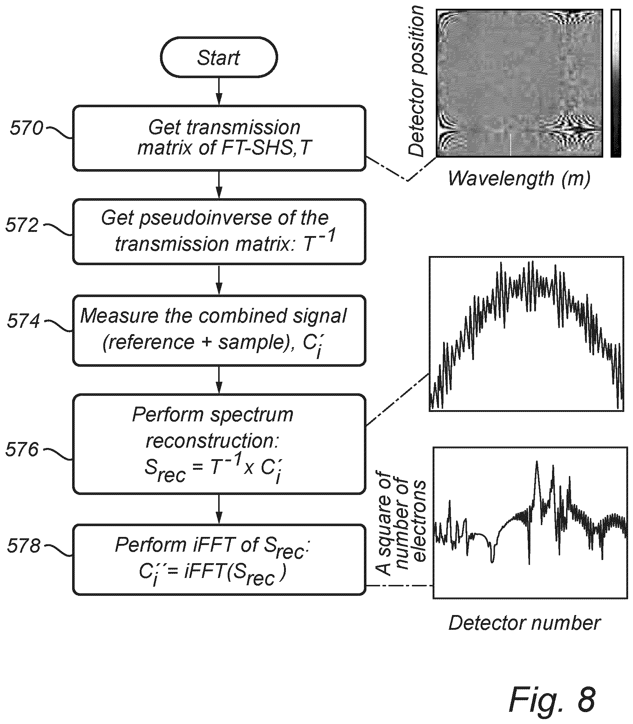

Referring now to Fig. 8, a calibration method used in spectroscopy may be incorporated into the FD-OCT system 500 to compensate for the signal distortion.

Initially, a transmission matrix T of the device 100, 200, 300 is determined 570, which converts a spectral domain signal to a transformation domain signal (i.e., the output signal provided by the photo-sensitive elements). This transmission matrix is illustrated in the graph associated with step 570.

It should be noted that the transmission matrix T encompasses all non-idealities such as dispersion and waveguide fabrication imperfections (e.g., variations in width and height). The transmission matrix $T$ may be obtained using a tunable laser and directly inputting an output from the tunable laser into the device 100, 200, 300 and performing measurements while tuning the laser. It should be noted that the tunable laser should have a tuning step $\delta z$ across the bandwidth $\Delta z$.

Thereafter, a pseudoinverse $T^{-1}$ of the transmission matrix (as $T$ is a nonsingular matrix) is computed 572. If $T$ is ill-conditioned (i.e., if small changes in the input cause large changes in the output), the Tikhonov regularization method may be used for computing the pseudoinverse. If $T$ is well-conditioned, the Moore-Penrose inverse may be used.

The pseudoinverse matrix $T^{-1}$ may then be used when processing measurements on samples. Thus, a measurement of a combined signal of the reference beam and the response beam from the sample may then be performed 574 by the device 100, 200, 300, producing output $C_i'$.

A spectrum $S_{rec}$ with reduced or removed impact of residual phase errors may then be reconstructed 576. This may be achieved by performing matrix multiplication of $T^{-1}$ and $C_i'$, such that $S_{rec} = T^{-1} \times C_i'$. The reconstructed spectrum is illustrated in the graph associated with step 576.

Finally, the calibrated signal $C_i''$ is obtained 578 by performing the inverse Fourier transform of $S_{rec}$. A corresponding processing may be performed for determining a calibrated reference signal $C_i$, which may then be subtracted from the calibrated signal $C_i''$.

Fig. 7 illustrates calibration results in a graph associated with step 578, wherein the calibration results are based on simulations, demonstrating effectiveness of the calibration process.

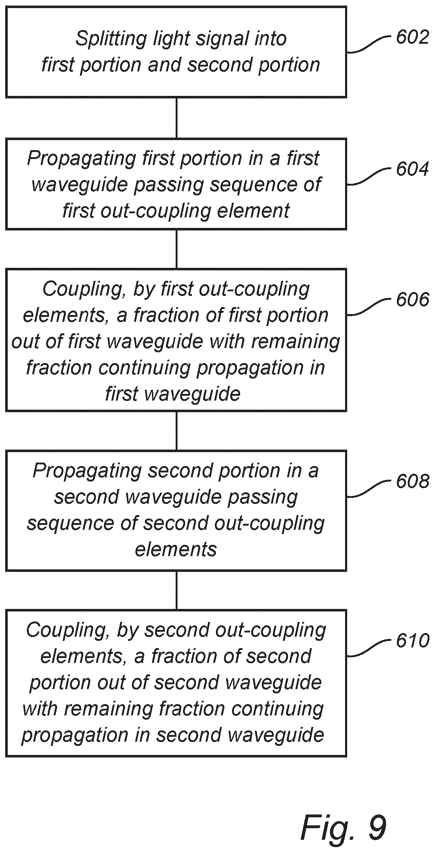

Referring now to Fig. 9, a method for guiding a light signal for Fourier Transform spectroscopy will be briefly described.

The method comprises splitting 602 a light signal into a first portion and a second portion. The light signal may for example be a signal carrying information of a sample.

The method further comprises propagating 604 the first portion of the light signal in a first waveguide passing a sequence of first out-coupling elements and coupling 606, by first out-coupling elements in the sequence, a fraction of the first portion of the light signal out of the first waveguide with a remaining fraction of the first portion of the light signal continuing propagation along the first waveguide. Thus, fractions of the first portion of the light signal may be simultaneously output by the first out-coupling elements.

The method further comprises propagating 608 the second portion of the light signal in a second waveguide passing a sequence of second out-coupling elements and coupling 610, by second out-coupling elements in the sequence, a fraction of the second portion of the light signal out of the second waveguide with a remaining fraction of the second portion of the light signal continuing propagation along the second waveguide. Thus, fractions of the second portion of the light signal may be simultaneously output by the second out-coupling elements.

It should be understood that the steps 604 and 606 are performed simultaneously with steps 608 and 610 by the first light propagation unit and the second light propagation unit, respectively.

An optical path length along the second waveguide between sequential second out-coupling elements is different than an optical path length along the first waveguide between corresponding sequential first out-coupling elements. The first out-coupling elements and second out-coupling elements form a sequence of pairs, each comprising one second out-coupling element and one corresponding first out-coupling element. A difference in the optical path length propagated by the fraction of the second portion of the light signal out-coupled by the second out-coupling element compared to the optical path length propagated by the fraction of the first portion of the light signal out-coupled by the corresponding first out-coupling element is configured to be different along the sequence of pairs.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

**1.** A device (100; 200; 300) for Fourier Transform spectroscopy, said device comprising:

an input waveguide (110; 210; 310) for receiving a light signal;
a splitter (112; 212; 312) connected to the input waveguide for splitting the light signal into a first portion and a second portion;
a first light propagating unit (120; 220; 320) configured to receive the first portion of the light signal, wherein the first light propagating unit (120; 220; 320) comprises a first waveguide (122; 222; 322) configured to propagate the first portion of the light signal, and a sequence of first out-coupling elements (124; 224; 324), wherein the first out-coupling elements (124; 224; 324) are spaced apart along an extension of the first waveguide (122; 224; 324), wherein first out-coupling elements (124; 224; 324) in the sequence are configured to couple a fraction of the first portion of the light signal out of the first waveguide (122; 222; 322) with a remaining fraction of the first portion of the light signal continuing propagation along the first waveguide (122; 222; 322);
a second light propagating unit (130; 230; 330) configured to receive the second portion of the light signal, wherein the second light propagating unit (130; 230; 330) comprises a second waveguide (132; 232; 332) configured to propagate the second portion of the light signal, and a sequence of second out-coupling elements (134; 234; 334), wherein the second out-coupling elements (134; 234; 334) are spaced apart along an extension of the second waveguide (132; 232; 332), wherein second out-coupling elements (134; 234; 334) in the sequence are configured to couple a fraction of the second portion of the light signal out of the second waveguide (132; 232; 332) with a remaining fraction of the second portion of the light signal continuing propagation along the second waveguide (132; 232; 332);
wherein an optical path length along the second waveguide (132; 232; 332) between sequential second out-coupling elements (134; 234; 334) is different than an optical path length along the first waveguide (122; 232; 332) between corresponding sequential first out-coupling elements (124; 234; 334), wherein the first out-coupling elements (124; 224; 324) and second out-coupling elements (134; 234; 334) form a sequence of pairs, each comprising one second out-coupling element (134; 234; 334) and one corresponding first out-coupling element (124; 224; 324), wherein a difference in the optical path length propagated by the fraction of the second portion of the light signal out-coupled by the second out-coupling element (134; 234; 334) compared to the optical path length propagated by the fraction of the first portion of the light signal out-coupled by the corresponding first out-coupling element (124; 224; 324) is configured to be different along the sequence of pairs.

2. The device according to claim 1, wherein the first out-coupling elements (124; 224; 324) in the sequence of first out-coupling elements (124; 224; 324) are configured to couple out an increasing fraction of the light signal from the first waveguide (122; 222; 322) in the sequence, and wherein the second out-coupling elements (134; 234; 334) in the sequence of second out-coupling elements (134; 234; 334) are configured to couple out an increasing fraction of the light signal from the second waveguide (132; 232; 332) in the sequence.

3. The device according to claim 1 or 2, wherein the optical path length along the second waveguide (132; 232; 332) between sequential second out-coupling elements (134; 234; 334) is larger than the optical path length along the first waveguide (122; 222; 322) between corresponding sequential first out-coupling elements (124; 224; 324), wherein the difference in the optical path length propagated by the fraction of the second portion of the light signal out-coupled by the second out-coupling element (134; 234; 334) compared to the optical path length propagated by the fraction of the first portion of the light signal out-coupled by the corresponding first out-coupling element (124; 224; 324) is configured to increase along the sequence of pairs.

4. The device according to claim 3, wherein the differences in optical path lengths is configured to increase along the sequence of pairs by an equal added path length between sequential pairs.

5. The device according to any one of the preceding claims, wherein out-coupling of the fraction of the first portion of the light signal and out-coupling of the fraction of the second portion of the light signal by each pair of first and second out-coupling elements is configured to be received directly in a common detection waveguide (326).

6. The device according to any one of claims 1-4, wherein each of the first out-coupling elements (224) comprises a first directional coupler configured to couple the fraction of the first portion of the light signal into a first detection waveguide (226), and wherein each of the second out-coupling elements (234) comprises a second directional coupler configured to couple the fraction of the second portion of light into a second detection waveguide (236).

7. The device according to claim 6, wherein the first detection waveguide (226) and the second detection waveguide (236) associated with a pair of the first out-coupling element (224) and the second out-coupling element (234) are connected to a combiner coupler (250) for forming a combined signal based on interference by the fraction of the first portion of the light signal and the fraction of the second portion of the light signal.

8. The device according to any one of claims 1-4, wherein each of the first out-coupling elements (124) comprises a first grating coupler configured to direct the fraction of the first portion of the light signal out of the first waveguide (122) and wherein each of the second out-coupling elements (134) comprises a second grating coupler configured to direct the fraction of the second portion of the light signal out of the second waveguide (132), wherein the first grating coupler and the second grating coupler are configured to direct the fraction of the first portion of the light signal and the fraction of the second portion of the light signal onto a common photo-sensitive area (142).

9. The device according to any one of the preceding claims, further comprising a plurality of photo-sensitive elements (140; 240; 340), wherein each photo-sensitive element (140; 240; 340) is configured to receive a combined signal formed by interference of the fraction of the first portion of the light signal and the fraction of the second portion of the light signal.

10. A system (400) for Fourier Transform spectroscopy, said system (400) comprising:

a polarization splitter (402) for splitting a combined polarization signal into a first light signal having a first polarization and a second light signal having a second polarization;
a first device (404) according to any one of the preceding claims, wherein the light signal received by the first device (404) is based on the first light signal; and
a second device (408) according to any one of the preceding claims, wherein the light signal received by the second device (408) is based on the second light signal.

11. The system according to claim 10, further comprising a polarization rotator (406) configured to receive the second light signal and configured to provide a rotation of the second polarization for forming the light signal received by the second device (408).

12. A system (500) for spectral domain interferometric measurement, said system comprising:

a light source (502) configured to provide illumination light;
a coupler element (504) configured to split the illumination light into a reference beam and a sample beam, wherein the system (500) is configured to output the sample beam towards a sample for interacting with the sample beam to form a response beam carrying information of the sample;
a device (100; 200; 300) according to any one of claims 1-9, wherein the light signal received by the device (100; 200; 300) is formed by a combination of reference beam and the response beam.

13. The system according to claim 12, said system (500) being configured to perform a reference measurement, wherein the light signal received by the device (100; 200; 300), during the reference measurement, is formed by the reference beam.

14. The system according to claim 12 or 13, wherein the system (500) is configured to output the sample beam towards biological tissue, such as a retina, for performing Optical Coherence Tomography, OCT.

15. A method for guiding a light signal for Fourier Transform spectroscopy, said method comprising:

splitting (602) a light signal into a first portion and a second portion;
propagating the first portion of the light signal in a first waveguide passing a sequence of first out-coupling elements;
coupling (604), by first out-coupling elements in the sequence, a fraction of the first portion of the light signal out of the first waveguide with a remaining fraction of the first portion of the light signal continuing propagation along the first waveguide;
propagating (606) the second portion of the light signal in a second waveguide passing a sequence of second out-coupling elements;
coupling (608), by second out-coupling elements in the sequence, a fraction of the second portion of the light signal out of the second waveguide with a remaining fraction of the second portion of the light signal continuing propagation along the second waveguide;

wherein an optical path length along the second waveguide between sequential second out-coupling elements is different than an optical path length along the first waveguide between corresponding sequential first out-coupling elements, wherein the first out-coupling elements and second out-coupling elements form a sequence of pairs, each comprising one second out-coupling element and one corresponding first out-coupling element, wherein a difference in the optical path length propagated by the fraction of the second portion of the light signal out-coupled by the second out-coupling element compared to the optical path length propagated by the fraction of the first portion of the light signal out-coupled by the corresponding first out-coupling element is configured to be different along the sequence of pairs.

Fig. 1

*Fig. 2a*

*Fig. 2b*

*Fig. 2c*

*Fig. 3a*

*Fig. 3b*

*Fig. 4a*

Fig. 4b

Fig. 4c

TE/TM

TE

TM

TE

400

404

402 406 408

## Fig. 5

λ

502

504

506

100,200,300

508

10

500

## Fig. 6

Fig. 7

Fig. 8

Splitting light signal into
first portion and second portion — 602

Propagating first portion in a first
waveguide passing sequence of
first out-coupling element — 604

Coupling, by first out-coupling
elements, a fraction of first portion
out of first waveguide with remaining
fraction continuing propagation in
first waveguide — 606

Propagating second portion in a
second waveguide passing
sequence of second out-coupling
elements — 608

Coupling, by second out-coupling
elements, a fraction of second
portion out of second waveguide
with remaining fraction continuing
propagation in second waveguide — 610

*Fig. 9*

Europäisches
Patentamt

European
Patent Office

Office européen
des brevets

# EUROPEAN SEARCH REPORT

**Application Number**

EP 24 21 9121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/167370 A1 (MASSACHUSETTS INST TECHNOLOGY [US]; KITA DEREK [US] ET AL.) 20 August 2020 (2020-08-20) * figure 5 * ----- | 1,15 | INV. G01J3/02 G01J3/453 ADD. G01B9/02091 |
| X | B. IMRAN AKCA: "Design of a compact and ultrahigh-resolution Fourier-transform spectrometer", OPTICS EXPRESS, vol. 25, no. 2, 19 January 2017 (2017-01-19), page 1487, XP055437986, DOI: 10.1364/OE.25.001487 | 1-9,15 | |
| Y | * figures 1,2c * * page 4, last paragraph * ----- | 10,11 | |
| X | WO 2018/055605 A1 (IXA AMC OFFICE / ACAD MEDICAL CENTER [NL]) 29 March 2018 (2018-03-29) * figures 1,4 * * paragraphs [0094], [0095] * ----- | 1-3,5-9, 12-15 | |
| A | GÜNAY YURTSEVER ET AL: "Ultra-compact silicon photonic integrated interferometer for swept-source optical coherence tomography", OPTICS LETTERS, OPTICAL SOCIETY OF AMERICA, US, vol. 39, no. 17, 1 September 2014 (2014-09-01), pages 5228-5231, XP001591572, ISSN: 0146-9592, DOI: 10.1364/OL.39.005228 [retrieved on 2014-08-29] * page 2, left-hand column, line 3 - line 7 * ----- -/-- | 10,11 | TECHNICAL FIELDS SEARCHED (IPC) G01J G01B |

The present search report has been drawn up for all claims

| Place of search | Date of completion of the search | Examiner |
|---|---|---|
| Munich | 28 April 2025 | Jacquin, Jérôme |

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 21 9121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2024/062509 A1 (PHOTONPATH S R L [IT]) 28 March 2024 (2024-03-28) * page 6, last paragraph - page 7, paragraph 2 * ----- | 10,11 | |

**TECHNICAL FIELDS SEARCHED (IPC)**

The present search report has been drawn up for all claims

| Place of search | Date of completion of the search | Examiner |
|---|---|---|
| Munich | 28 April 2025 | Jacquin, Jérôme |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 9121

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2025

| Patent document cited in search report | | Publication date | Patent family member(s) | | Publication date |
|---|---|---|---|---|---|
| WO 2020167370 | A1 | 20-08-2020 | US | 2020256728 A1 | 13-08-2020 |
| | | | US | 2021239526 A1 | 05-08-2021 |
| | | | WO | 2020167370 A1 | 20-08-2020 |
| WO 2018055605 | A1 | 29-03-2018 | CN | 110312918 A | 08-10-2019 |
| | | | EP | 3516354 A1 | 31-07-2019 |
| | | | KR | 20200005524 A | 15-01-2020 |
| | | | US | 2020025616 A1 | 23-01-2020 |
| | | | WO | 2018055605 A1 | 29-03-2018 |
| WO 2024062509 | A1 | 28-03-2024 | NONE | | |

EPO FORM P0459

For more details about this annex : see Official Journal of the European Patent Office, No. 12/82